# EUROPEAN PATENT SPECIFICATION

(11) **EP 3 834 769 B1**
(45) Date of publication and mention of the grant of the patent: **25.01.2023**
(21) Application number: 20203766.9
(22) Date of filing: 26.10.2020
(51) Int. Cl.: A61C 1/00, A61C 1/08

(54) **PORTABLE DEVICE FOR ROBOTIC INTRAORAL PREPARATION OF ABUTMENT TEETH**
TRAGBARE VORRICHTUNG FÜR DIE ROBOTISCHE INTRAORALE PRÄPARATION VON ABUTMENTZÄHNEN
DISPOSITIF PORTABLE POUR LA PRÉPARATION ROBOTIQUE INTRAORALE DES DENTS DU PILIER

(43) Date of publication of application: 16.06.2021
(73) Proprietor: Fruman, Radu-Ioan, Brasov (RO)
(72) Inventor: Fruman, Radu-Ioan, Brasov (RO)
(74) Representative: Bozocea, Gina Violeta

(56) References cited:
- WO-A1-94/03120
- WO-A1-2020/105052
- US-B2- 9 554 872

## Description

The invention relates to a portable device for the robotic intraoral preparation of abutment teeth for the immediate cementation of partial or total prosthetic restorations, previously performed on virtual models.

A dental work generally aims to restore functionality and / or aesthetics and can be performed in any intraoral area, and to prepare a tooth for placement of a work, the dentist must work on the construction of the abutment teeth. Thus, after the milling of the abutment teeth, a impression of the abutment teeth is made, which is thus prepared and sent to a laboratory to carry out the respective work.

Various robotic equipments used in the field of dentistry are known from the technical step. Various computer-generated templates are also known for use with these robotic devices.

US 4182312 A discloses a dental probe having a stylus which is connected through a rod to a three position transducer which produces three signals indicating the position of the probe at any point to which the probe is applied. The transducers are mounted on an index tray which is adapted to be fastened to the jaw of the patient. Thus the patient's jaw becomes the origin against which all measurements are made. A handle is connected to the probe in such a way that the dentist has three rotational degrees of freedom of movement without affecting the position of the end of the stylus. Contact between the tip of the stylus and the patient's tissue completes a circuit to turn on the recording mechanism which receives the transducers' outputs.

DE 4012327 A1 discloses a dental jaw model or similar undergoes an ideal preparation corresponding to the specified task and an analysis by a parallelometer, using a parallelometer grinding system. The preparation data are further transmitted to a computer and a 3-D milling unit and a grinding body, and servo motors are used with horizontal and vertical rotors. A milling template is adapted for positioning in the mouth of the patient, e.g. using a turbine grinder or a grinding body. Following the positioning and fixing of the template(s) in the mouth of the patient, the scanning of the grinding body is carried out through the milled out part of the template and the ideal preparation of the jaw model is transmitted to the natural teeth. Also supplied across the cable connections are rotary and linear potentiometers, photo-electric measurement systems and laser interferometer or similar as displacement and angle sensors.

From document US 2014242541 A1 a dental cutting system and method for cutting a patient's tooth are known. The dental cutting system includes a hand piece to be held and carrying a cutting burr to cut the patient's tooth. A plurality of guide pins is attached to and extends from the hand piece of the cutting tool remote from the cutting bur. One or more cutting guides each have a cavity coinciding with at least a portion of teeth inside a patient's mouth that are adjacent to or remote from the patient's tooth to be cut. The guide pins of the handpiece slide in slots in the cutting guide to guide the burr to cut the patient's tooth.

WO 2020/105052 A1 discloses a mountable dental device for shaping a tooth of a subject. The device includes: (i) an anchoring member configured to be removably affixed to a jaw of a subject by securing thereof to one or more teeth of the subject, and (ii) a computerized numerical control (CNC) machine fixedly mounted or mountable on the anchoring member. The CNC machine is configured to have installed thereon and maneuver a dental turbine. When the anchoring member is affixed to the jaw, the device is supported by the subject. The anchoring member is adjustable such as to allow the affixing thereof to jaws of different subjects. The CNC machine is configured to control operation of the dental turbine in at least one dental tooth-shaping procedure on at least one tooth of the subject.

US 9554872 B2 relates to an intraoral device for automated preparation of the teeth with a view to performing partial or peripheral dental restoration, which includes: a splint suitable for being positioned in the mouth of a patient, said splint including a means for maintaining the position thereof inside said mouth; at least one mobile cutting tool combined with said splint; and an electronic management unit which makes it possible to control said cutting tool, characterised in that: said cutting tool is configured so as to cut at least the labial surface of the tooth to be prepared, said tool being mounted on a mobile carriage moving along a rail attached to the splint, opposite the labial surface of the tooth to be prepared, said rail having a curvature that matches the dentition of the patient; said splint includes at least one 3D digitisation tool arranged such as to digitise at least the labial surface of said tooth to be prepared, said digitisation tool being connected to the management unit so that the digitised data can be transferred to said management unit; and said management unit is configured so as to control the movement of said cutting tool in accordance with the digitised data.

WO 94/03120 A1 discloses a dentistry assembly having a dental tool connected by a releasable coupling to a handle. The handle is adjustable in position relative to the drill body to arrange the tool head at a desired angle for operating on a patient's tooth. the dentist may position a tool in a desired location relative to a patient's tooth and it will always remain in the desired controlled position.

The present invention aims to provide a portable device for the robotic intraoral preparation of abutment teeth, which completely eliminates the talent and experience factors associated with the doctor performing the operation, and which allows an accurate, clear and correct realisation of abutment teeth, for a prefabricated job through a method that removes the imprinting of the prepared abutment teeth.
The solution to this problem is a portable device for robotic intraoral preparation of abutment teeth **and a milling guide, the portable device comprising a turbine head,** a handle provided at the top with a stepper motor for each of the X, Y and Z axes, so that it is possible moving a turbine head along guide rails on each of the X, Y and Z axes, wherein the device comprises a fixing rail mounted at one **of the ends of the fixing rail** in the handle of the device, and at the free end the fixing rail is provided with a rectangular frame, the fixing rail extending to the lower part and parallel to the arm of the turbine head, so that **a** bur mount**able** in the turbine head is movably controll**able** inside the frame of the fixing rail, wherein the frame is mountedable by means of fixing screws in threaded bushes **on the milling guide,** in correspondence with an opening of the milling guide, which is fix**able** in advance on the teeth by retentivity and intraosseous by means of pins.

In a preferred embodiment of the device according to the present invention, the bur is **movably controllable** on the basis of a software for making the shape of abutments designed virtually in the laboratory, the movement inside the frame being guided on the basis of fixing bushes.

Preferably, the device according to the present invention comprises an emergency stop button.

In an advantageous way, the device according to the present invention comprises feeding means with sterile physiological serum, with cooling role, arranged in the turbine head.

The present invention has the following advantages and contributes to the elimination of aspects related to:
- Subjective factors that relate to the skill and experience of the doctor,
- Excessive grinding of the abutment teeth,
- The unexpected final result, which is unpleasantly identified with various unacceptable "technical" explanations, such as: lack of interdental space, defective bite, urgency of the procedure, late hour etc.
- The realisation by the technician of some unwanted compromises, corrections and artifices in order to mask the doctor's errors in the preparation of the abutment teeth, which will lead to the accomplishment of some unsightly works, incorrectly performed, with short life,
- The use of materials and methods of classical imprinting, with the associated increase of the total time of accomplishing the dental work,
- Any type of imprinting of the prepared abutment teeth, thus eliminating the inaccuracies that are transmitted to the laboratory
- The at least 7 visits during at least 3 weeks to obtain the final works in the classic version
- Large irregularities regarding the space between the column and the dental crown, which lead to very different thicknesses of the cement layer used in the prosthetic work, which implicitly in time leads to its fracturing and to the partial or total decimation of the works.

Other objects, features and advantages of the present invention will become more apparent from the following detailed description of a preferred embodiment, given by way of non-limiting example, of the portable device for the robotic intraoral preparation of abutment teeth, in connection with the appended figures, wherein :
Fig. 1 is an overview of the portable device for the robotic intraoral preparation of abutment teeth, according to the present invention;
Fig. 2 is an overview of the portable device for the robotic intraoral preparation of the abutment teeth, according to the present invention, in the working position;
Fig. 3 and 4 are detailed views of the position of the frame for fixing the portable device for the robotic intraoral preparation of the abutment teeth, according to the present invention, in relation to the milling guide;
Fig. 5 is an overview of a simplified example of a milling guide made of transparent resin;

In connection with the appended figures, and for a clearer and more comprehensive understanding of the present invention, the entire sequence of the steps for preparing the dental work in which the portable device for the robotic intraoral preparation of the abutment teeth is used according to the present invention will be further presented.

In a first step, the photos are taken with the patient's smile, with a simple camera, ideally with a white background and the introduction of images in a existing software for simulating dental treatment - a step made in the dental office.

Following next is the virtual simulation of the future smile - Virtual mock-up and the presentation of the materials from which the work can be done, the costs, the patient's agreement for the shape, colour, and cost. This step is performed in the clinic or laboratory.

Coronary reconstruction is then performed by specific methods known when appropriate (step performed in the office), and intraoral scanning of both arches, as well as occlusion with a known intraoral scanner (step performed in the clinic), after which the images and intraoral scanning (step performed in the office) are sent to the laboratory / doctor.

Next, the laboratory / doctor creates a virtual mock-up (virtual volumetric simulation of the future work), in a specific program, existing in the laboratory (step performed in the laboratory).

Then the printing of a model after mock-up and the realisation of a temporary work milled from a temporary material, which simulates the final work (step performed in the laboratory).

The temporary intraoral work is cemented with dental bonding or resin cement (step performed in the clinic).

Depending on the clinical context, if necessary, in the same session or after about 2 days, the temporary work will be adapted to the patient's usual occlusion, as well as to the patient's aesthetic demands (step performed in the office).

After the patient's consent for the final work - form + material, there is an intraoral scan of the arch or arches which will be replaced by prosthetics, as well as the occlusion with the temporary intraoral work, and the removal or not of the temporary work, according to the patient's wish (step performed in clinic).

Intraoral scanning data and the creation of a milling guide through an existing dental program are then transmitted to the laboratory. The milling guide can be made of a transparent resin for surgical guides, on the entire arch, with 4 recesses, with metal reinforcement, for gripping the rail of the device for robotic intraoral preparation of abutment teeth, as will be detailed below. The milling guide is provided with an opening in the area in which the prosthetic abutments / pillars are to be prepared and with 3-5 holes for the bone fixation of the guide with pins, intraorally.

In order to achieve the final work in the form and material agreed by the patient, the virtual reduction of each pillar in the model scanned with the temporary work, with a shape and thickness specific to the material from which the final work is to be performed, in an existing dental program, and at the end of this step, a virtual model will be obtained with the abutments on which the final work is to be based (the step is performed in the laboratory).

In the situation where there are vital pillar teeth, the need for their devitalisation will be analysed and decided, following the realisation of the devitalisations and canal fillings considered to be necessary (step performed in the clinic).

Then the printing and analysis of the model with the abutments to be obtained is performed and the virtual realisation of the final work on the virtual model obtained (step performed in the laboratory).

The final work is tested on the printed model and the milling guide is fixed on the printed or cast model of the temporary work, the device for robotic intraoral preparation is fixed on the milling guide and the verification/simulation, with an active diamond bur on the material device, of the abutment preparation with the help of the device, on the model. Then the verification of the adaptation of the final work on the model thus obtained takes place (step performed in the laboratory).

Anaesthesia of the areas to be prepared takes place and the intraoral milling guide is fixed on the existing teeth to be prepared and, if necessary, intraosseous by means of pins, and the calibration of the robotic intraoral preparation of the abutment teeth on the guide and the mounting of the occlusal stops. In the situation that the work has a size exceeding 6 teeth, then it is necessary to make two milling guides, the first for 1 hemiarcade, according to the initial situation, and the second one based on the situation obtained after using the first guide and preparing the abutment teeth.
the bur to be used together with the portable device for robotic intraoral preparation of abutment teeth is mounted, according to the present invention, the verification of the milling rotation settings as well as the operation of the cooling irrigation takes place. The fixing rail attaches to the milling guide and then the portable device is operated for the robotic intraoral preparation of the abutment teeth.

After preparation, the intraoral work is tested and the final or provisional cementing takes place, if required.

Going back to the accompanying figures, the portable device 1 for the robotic intraoral preparation of the abutment teeth, according to the present invention, comprises a handle 11 provided at the top with a stepper motor 7, 9, 14 for each of the X, Y and Z, so that it is possible to move a turbine head 3 along guide rails 5, 6, 10 on each of the X, Y and Z axes.

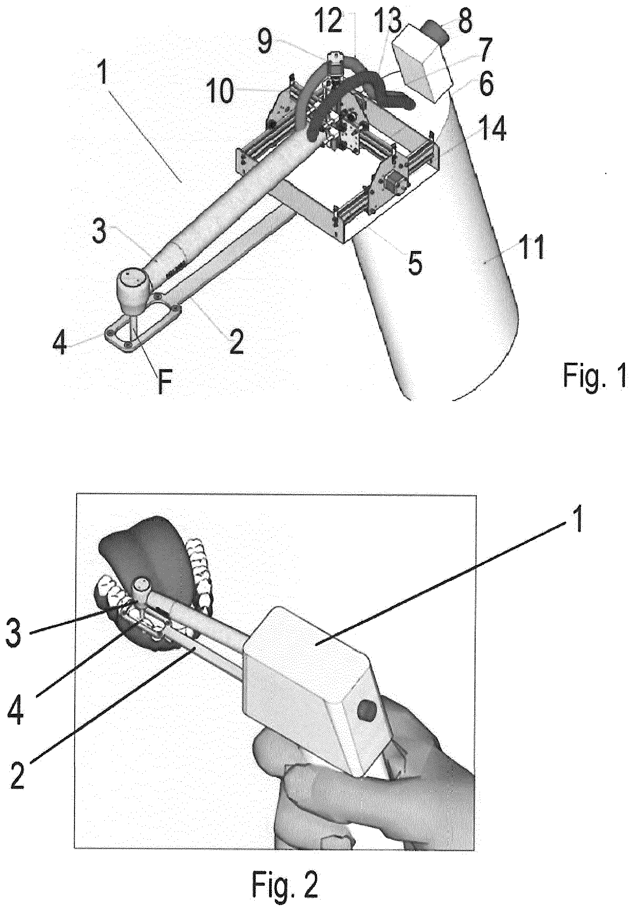

The device according to the invention comprises a fixing rail 2 mounted at one of the ends in the handle 11 of the device, and at the free end provided with a rectangular frame 4, the fixing rail 2 extending to the lower part and parallel to the turbine head arm 3, so that the bur F mounted in the turbine head 3 is controlled movable inside the frame 4 of the fixing rail 2. the bur is driven, in a known manner, with compressed air (see compressed air supply hose 13 in figure 1).

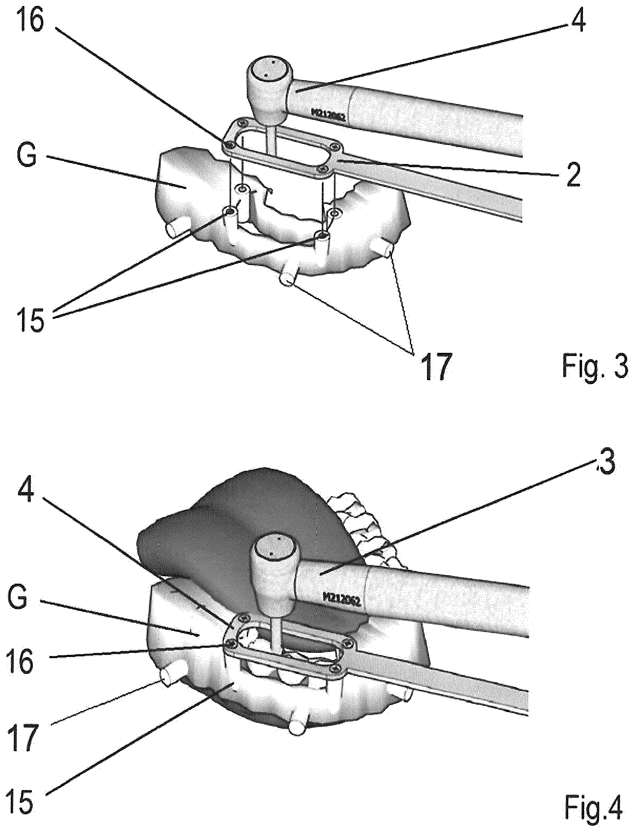

With particular reference to Figures 3 and 4, the frame 4 is attached / mounted to the threaded bushes 15 by means of fixing screws 16, in correspondence with an opening L of a milling guide G, which is fixed in advance on the teeth by retentivity and intraosseous by means of pins 17.

According to the invention, the milling machine is controlled movable on the basis of a software for realising the shape of the abutments designed virtually in the laboratory, the movement inside the frame 4 being guided on the basis of the fixing bushes 15 which constitute landmarks for moving the bur.

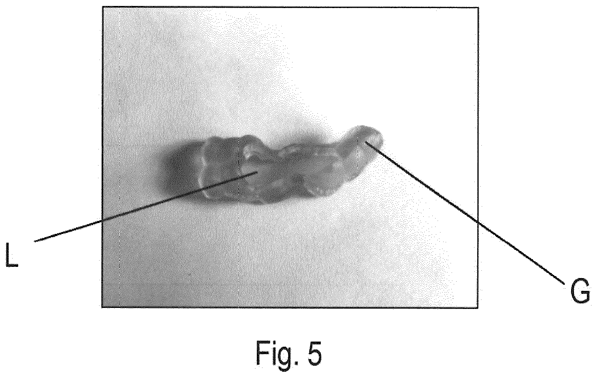

With reference to Figure 5, and as mentioned above, the milling guide G is made of a transparent resin and has an opening L in the area in which the prosthetic abutments / columns are to be prepared.

Advantageously, the device according to the invention comprises a stop button 8 in case of emergency.

Also, as mentioned above, the device according to the invention comprises feeding means 12 with sterile physiological serum, with cooling role arranged in the turbine head 3.

One skilled in the art will appreciate that the invention has been disclosed above in an illustrative and non-limiting manner, and that numerous modifications and changes may be made within the scope of the invention without departing from the scope of the invention as defined in the appended claims.

## Claims

1. A portable device for robotic intraoral preparation of abutment teeth and a milling guide (G), the portable device comprising a turbine head, a handle (11) provided at the top with a stepper motor (7, 9, 14) for each of the X, Y and Z axes, so that it is possible to move the turbine head (3) along guide rails (5, 6, 10) on each of the X, Y and Z axes, wherein the device comprises a fixing rail (2) mounted at one of its ends in the handle (11) of the device, and at a free end the fixing rail is provided with a rectangular frame (4), the fixing rail (2) extending to a lower part and parallel to an arm of the turbine head (3), so that a bur (F) mountable in the turbine head (3) is movably controllable inside the frame (4) of the fixing rail (2), **characterized in that** the frame (4) is mountable by means of fixing screws (16) in threaded bushes (15) on the milling guide, in correspondence with an opening (L) of a the milling guide (G), which is fixable in advance on the teeth by retentivity and intraosseous by means of pins (17).

2. The device according to claim 1, **characterized in that** the bur (F) is movably controllable on the basis of a software for making the shape of abutments designed virtually in the laboratory, the movement inside the frame (4) being guided on the basis of the fixing bushes (15) which constitute diamond bur movement marks

3. The device according to Claim 1 or 2, **characterized in that** said frame (4) is rigidly attachable to the milling guide (G).

4. The device according to one of Claims 1 to 3, **characterized in that** the milling guide (G) is made of a transparent resin having said opening (L) in the area in which the prosthetic abutments / columns are to be prepared.

5. The device according to one of Claims 1 to 4, **characterized in that** it comprises an emergency stop button (8).

6. The device according to one of Claims 1 to 5, **characterized in that** it comprises means for supplying (12) a sterile physiological serum with cooling role, arranged in the turbine head (3).

## Patentansprüche

1. Eine tragbare Vorrichtung zur robotergestützten intraoralen Präparation von Pfeilerzähnen und eine Fräsführung, wobei die tragbare Vorrichtung einen Turbinenkopf, einen Griff (11), der an der Oberseite mit einem Schrittmotor (7, 9, 14) für jede der X-, Y- und Z-Achsen versehen ist, umfasst, so dass es möglich ist, einen Turbinenkopf (3) entlang von Führungsschienen (5, 6, 10) auf jeder der X-, Y- und Z-Achsen zu bewegen, wobei die Vorrichtung eine Befestigungsschiene (2) umfasst, die an einem der Enden der Befestigungsschiene im Griff (11) der Vorrichtung angebracht ist, und die Führungsschiene am freien Ende mit einem rechteckigen Rahmen (4) versehen ist, wobei sich die Befestigungsschiene (2) bis zum unteren Teil und parallel zum Arm des Turbinenkopfes (3) erstreckt, so dass ein im Turbinenkopf (3) montierbarer Bohrer (F) innerhalb des Rahmens (4) der Befestigungsschiene (2) beweglich steuerbar ist, **dadurch gekennzeichnet, dass** der Rahmen (4) mittels Befestigungsschrauben (16) in Gewindebuchsen (15) auf der Fräsführung in Übereinstimmung mit einer Öffnung (L) der Fräsführung (G) montierbar ist, die im Voraus auf den Zähnen durch Remanenz und intraossär mittels Stiften (17) fixierbar ist.

2. Die Vorrichtung nach Anspruch 1, **dadurch gekennzeichnet, dass** der Bohrer (F) mittels einer Software zur Herstellung der Form von virtuell im Labor entworfenen Stützpfeiler beweglich gesteuert werden kann, wobei die Bewegung im Inneren des Rahmens (4) auf der Grundlage der Befestigungsbuchsen (15) geführt wird, die die Bewegungsmarkierungen für den Diamantbohrer bilden.

3. Die Vorrichtung nach Anspruch 1 oder 2, **dadurch gekennzeichnet, dass** der Rahmen (4) starr mit der Fräsführung (G) verbunden ist.

4. Die Vorrichtung nach einem der Ansprüche 1 bis 3, **dadurch gekennzeichnet, dass** die Fräsführung (G) aus einem transparenten Kunststoff hergestellt ist, der die Öffnung (L) in dem Bereich aufweist, in dem die prothetischen Stützpfeiler/Pfeiler vorbereitet werden sollen.

5. Die Vorrichtung nach einem der Ansprüche 1 bis 4, **dadurch gekennzeichnet, dass** sie einen Not-Aus-Knopf (8) umfasst.

6. Die Vorrichtung nach einem der Ansprüche 1 bis 5, **dadurch gekennzeichnet, dass** sie im Turbinenkopf (3) angeordnete Mittel zum Zuführen (12) eines sterilen physiologischen Serums mit Kühlfunktion umfasst.

## Revendications

1. Un dispositif portable pour la préparation intra-orale robotisée de dents piliers et un guide de fraisage, le dispositif portable comprenant une tête de turbine, une poignée (11) munie à sa partie supérieure d'un moteur pas à pas (7, 9, 14) pour chacun des axes X, Y et Z, de sorte qu'il soit possible de déplacer une tête de turbine (3) le long de rails de guidage (5, 6, 10) sur chacun des axes X, Y et Z, dans lequel le dispositif comprend un rail de fixation (2) monté à l'une des extrémités du rail de fixation dans la poignée (11) du dispositif, et à l'extrémité libre le rail de guidage est muni d'un cadre rectangulaire (4), le rail de fixation (2) s'étendant vers la partie inférieure et parallèlement au bras de la tête de turbine (3), de telle manière qu'une fraise (F) pouvant être montée dans la tête de turbine (3) puisse être contrôlée de manière mobile à l'intérieur du cadre (4) du rail de fixation (2), **caractérisé en ce que** le cadre (4) peut être monté au moyen de vis de fixation (16) dans des douilles filetées (15) sur le guide de fraisage, en correspondance avec une ouverture (L) du guide de fraisage (G), qui peut être fixé à l'avance sur les dents par rétention et à niveau intra-osseux à l'aide de broches (17).

2. Le dispositif selon la revendication 1, **caractérisé en ce que** la fraise (F) peut être contrôlée de manière mobile sur la base d'un logiciel de réalisation de la forme des piliers conçu virtuellement en laboratoire, le mouvement à l'intérieur du cadre (4) étant guidé au moyen des douilles de fixation (15) qui constituent des repères de mouvement de la fraise diamantée.

3. Le dispositif selon la Revendication 1 ou 2, **caractérisé en ce que** ledit cadre (4) peut être fixé de manière rigide au guide de fraisage (G).

4. Le dispositif selon l'une des revendications 1 à 3, **caractérisé en ce que** le guide de fraisage (G) est fait d'une résine transparente présentant ladite ouverture (L) dans la zone dans laquelle les piliers / colonnes prothétiques doivent être préparés.

5. Le dispositif selon l'une des revendications 1 à 4, **caractérisé en ce qu'**il comprend un bouton d'arrêt d'urgence (8).

6. Le dispositif selon l'une des revendications 1 à 5, **caractérisé en ce qu'**il comprend des moyens pour fournir (12) un sérum physiologique stérile ayant un rôle de refroidissement, disposés dans la tête de turbine (3).
